**Europäisches Patentamt**

**European Patent Office**

**Office européen des brevets**

(19)

(11) Publication number: **0 090 433**
**A1**

(12) # EUROPEAN PATENT APPLICATION

(21) Application number: **83103253.7**

(22) Date of filing: **31.03.83**

(51) Int. Cl.³: **C 12 N 15/00,** C 12 P 21/02, C 07 C 103/42, C 12 P 19/34, C 07 H 21/00

(30) Priority: **31.03.82 US 363657**

(43) Date of publication of application: **05.10.83**
**Bulletin 83/40**

(84) Designated Contracting States: **AT BE CH DE FR GB IT LI LU NL SE**

(71) Applicant: **Genetics Institute, 225 Longwood Avenue, Boston Massachusetts 02115 (US)**

(72) Inventor: **Seed, Brian S., 7 Divinity Avenue, Cambridge Massachusetts (US)**

(74) Representative: **Berg, Wilhelm, Dr. et al, Dr. Wilhelm Berg, Dipl.-Ing. Otto F. Stapf Dipl.-Ing. Hans-Georg Schwabe Dr. Dr. Kurt Sandmair, Patent Attorneys Mauerkircherstrasse 45, D-8000 Munich 80 (DE)**

(54) **Creation of DNA sequences encoding modified proinsulin precursors.**

(57) Methods and compositions are provided for the production of insulin. DNA sequences encoding modified proinsulin precursors are produced in vitro and inserted into production cells wherein modified proinsulin precursors are elaborated. The modified proinsulin precursors are cleaved in vitro to yield insulin. Modified proinsulin precursors comprise aspartic acid at the amino terminus of the C chain and/or methiorine at the carboxyl terminus of the C chain.

EP 0 090 433 A1

## CREATION OF DNA SEQUENCES ENCODING MODIFIED
## PROINSULIN PRECURSORS

### Background of the Invention

Insulin is manufactured in the pancreas initially
as pre-proinsulin which is cleaved to form proinsulin.
Proinsulin contains three amino acid segments, A and B
chains which form the insulin molecule and a C chain
connecting the A and the B chains.  In vivo, the C chain
is cleaved to yield insulin by enzymes that have not yet
been identified.

The synthetic production of insulin has presented
numerous problems.  The DNA sequence encoding proinsulin
can be inserted into a vector and placed into production
cells wherein proinsulin is expressed.  However, without
the specific enzymes, in vivo cleavage of the C chain
does not, occur.  The C chain may be cleaved in vitro, but
additional cleavages of the A and B chain also occur,
significantly reducing the yield of insulin by this
method.

The A and B amino acid chains may be separately
synthesized and then bonded to one another without the
C chain.  This has the drawback that the A and B chains
must be separately purified.  In addition, the A and B
chains frequently align themselves into incorrect
configurations without the C chain.

## Summary of the Invention

There are provided compositions and methods for the production of insulin. According to the present invention, insulin is produced by a process comprising the in vivo synthesis of a modified proinsulin precursor. Modified proinsulin precursors comprise proinsulin precursors comprising proinsulin having an amino acid substitution selected from the group consisting of aspartic acid substituted for the amino acid at the amino terminus of the C chain, methionine substituted for the amino acid at the carboxyl terminus of the C chain, and combinations thereof. The preferred proinsulin precursor comprises human proinsulin.

A DNA sequence encoding modified proinsulin precursors is prepared by annealing at least one select oligonucleotide primer to a vector containing a single-stranded DNA sequence encoding a proinsulin precursor. In a preferred embodiment, the primer is annealed to a single-stranded complementary DNA (cDNA) encoding a proinsulin precursor comprising human proinsulin that has been inserted into an M13 vector.

To effect the substitution of aspartic acid for the amino acid at the amino terminus of the C chain, the primer contains a nucleotide triplet encoding aspartic acid and sufficient nucleotides complementary to a single-stranded, message-synonymous DNA sequence encoding the proinsulin precursor to effectively anneal the primer to the single-stranded DNA sequence at a position wherein the aspartic acid encoding triplet encodes the amino acid at the carboxyl terminus of the C chain.

Likewise, to substitute aspartic acid for the amino acid at the amino terminus of the C chain, a primer that is the complement of the above-described primer is prepared and annealed to a single-stranded coding DNA sequence encoding the proinsulin precursor.

To effect the substitution of methionine for the amino acid at the carboxyl terminus of the C chain, the primer contains a nucleotide triplet encoding methionine and sufficient nucleotides complementary to a single-stranded DNA sequence encoding the proinsulin precursor to effectively anneal the primer to the single-stranded, message-synonymous DNA sequence at a position wherein the methionine encoding triplet encodes the amino acid at the carboxyl terminus of the C chain.

Similarly, to substitute methionine for the amino acid at the carboxyl terminus of the C chain, a primer that is the complement to the above-described primer containing a triplet encoding methionine is annealed to the single-stranded coding DNA sequence encoding the proinsulin precursor.

To identify vectors containing an altered DNA sequence encoding modified proinsulin precursors following cloning, a nucleotide sequence containing a restriction enzyme recognition sequence is also annealed to the singlestranded, message-synonymous DNA sequence encoding the proinsulin precursor. Preferably, the primer contains such a restriction enzyme recognition sequence. Alternatively, a nucleotide sequence containing the complement of a restriction enzyme recognition sequence is annealed to the coding strand of the DNA sequence.

After the primer and, if needed, a nucleotide sequence containing a restriction recognition sequence or its complement have been annealed to the vector, the primer is extended and ligated to form a circular double-stranded vector which is then inserted into cells such as E. coli. The cells and the vectors replicate. This forms at least two groups of vectors, one group comprising an unaltered DNA sequence encoding normal proinsulin and a second group comprising an altered DNA sequence encoding modified proinsulin precursors. The vectors carrying the altered DNA sequence can be identified by conventional techniques such as electrophoresis following digestion with the restriction enzyme and are then isolated.

The altered DNA sequence is then cleaved from the vector and inserted into a second vector capable of expressing high levels of proinsulin in production cells.

If modified proinsulin precursors containing both amino acid substitutions are desired, two primers, each containing a nucleotide triplet capable of effecting one of the desired substitutions or its complement, may be annealed simultaneously or in sequence to the single-stranded DNA sequence encoding a proinsulin precursor in either the (+) or (-) orientation.

It is preferred that the second primer be annealed in a successive step rather than simultaneously. After vectors containing an altered DNA sequence in which one primer has been incorporated have been isolated, one strand of the double-stranded altered DNA sequence of the vector is exposed.

The second primer and, if necessary, a second nucleotide sequence containing a restriction enzyme recognition sequence or its complement are annealed to the single-stranded altered DNA sequence and then extended and ligated. The double-stranded vector thus formed is again introduced into E. coli cells wherein the cells and vector replicate.

Vectors containing both DNA sequence alterations are then identified and isolated. The twice-altered DNA sequence encoding modified proinsulin precursors having both amino acid substitutions is then cleaved from the vector and inserted into a new vector expressing high levels of proinsulin precursors in a production cell of choice.

Modified proinsulin precursors produced in the cells are recovered and purified. A preferred general method for recovery and purification is by specific absorption to monoclonal antibodies attached to agarosebead chromographic media. The monoclonal antibodies are prepared to have a specific affinity for a particular fragment of the modified proinsulin precursor molecule. Following washing of the monoclonal antibody column, the modified proinsulin precursor is desorbed.

The C chain and amino acid chains attached to the amino terminus of the B chain and the carboxyl terminus of the A chain are cleaved from the modifed proinsulin precursor in vitro to yield insulin.

Insulin thus produced is purified by solvent fractionation, salt precipitation, specific absorption on a monoclonal antibody column and the like.

## Detailed Description

According to principles of the invention, insulin is produced by cleaving a modified proinsulin precursor in vitro. Proinsulin is composed of three amino acid chains, designated A, B and C. The C chain connects the A and B chain and is cleaved from the proinsulin to yield insulin, which is composed of the A and B chains. Proinsulin precursors comprise proinsulin and may contain an amino acid chain preceding the B chain, i.e., attached to the amino terminus of the B chain, and/or may contain an amino acid chain following the A chain, i.e., attached to the carboxyl terminus of the A chain.

By convention, the amino acids of proinsulin are labeled according to position starting at the amino terminus of the B chain. The method herein is applicable to all proinsulin precursors, particularly those comprising human proinsulin in which neither the B chain nor the A chain of proinsulin contain the amino acids methionine or aspartic acid.

Human proinsulin has a sequence of 86 amino acids. The B chain consists of the amino acids at positions 1 through 30. The C chain of human proinsulin consists of amino acids at positions 31 through 65 and the A chain consists of amino acids at positions 66 through 86. Cleavage of human proinsulin to form insulin occurs between the amino acids at positions 30 and 31 and between the amino acids at positions 65 and 66.

The lengths and compositions of the A and B chains of insulin are highly conserved evolutionarily, i.e., fairly constant from animal to animal. However, the length and composition of the C chain of proinsulin does vary widely according to the source.

A modified proinsulin precursor differs from such a proinsulin precursor in that the modified proinsulin precursor contains an amino acid substitution wherein aspartic acid is substituted for the amino acid at the amino terminus of the C chain, e.g., amino acid position 31 of human proinsulin; or methionine is substituted for the amino acid at the carboxyl terminus of the C chain, e.g., amino acid position 65 of human proinsulin; or aspartic acid is substituted for the amino acid at the amino terminus of the C chain and methionine is substituted for the amino acid at the carboxyl terminus of the C chain. In addition, at least one peptide chain may be present either attached to the amino terminus of the B chain or the carboxyl terminus of the A chain. The appended amino acid chains may either contain sequences allowing the in vivo cleavage of proinsulin, such as the native human proinsulin prepeptide, or the amino acid chain preceding the B chain may contain methionine at the -1 position of proinsulin, and/or the amino acid forming the amino terminus of the amino acid chain following the A chain may be aspartic acid, thus allowing cleavage in vitro.

In a preferred embodiment for the production of a modified proinsulin precursor, a complementary DNA (cDNA) sequence encoding a proinsulin precursor comprising human proinsulin is inserted into a double-stranded M13 vector. The cDNA is made by extracting RNA transcribed by naturally-occurring chromosomal DNA from human insulin-producing pancreatic cells and then making a DNA copy from the RNA.

The cDNA is inserted into a circular double-stranded M13 vector by first cleaving the M13 vector and the cDNA (leaving intact the gene encoding human proinsulin) with an endonuclease which forms cohesive termini at the cleaved ends of the M13 vector and the cDNA or by joining cohesive ends to the cDNA enzymatically. The cDNA is annealed to the vector by mixing, and, if necessary, ligated with DNA ligase, which catalyzes the formation of phosphate bonds between the cDNA and the vector.

E. coli cells are transformed with the M13 vectors by standard techniques. For example, the cells may be grown on a suitable medium to a particular optical density followed by concentrating the cells in a series of steps. The cells are suspended in a calcium chloride or manganese chloride/rubidium chloride solution at reduced temperatures (about 0°C to 5°C) and then mixed with the M13 vectors at the reduced temperature. The mixture is then heated to about 35°C to about 45°C for up to 5 minutes and again chilled. The cells are then transferred to a growth medium.

In the cell, the vector replicates. A portion of the double-stranded vectors form circular single-stranded vectors which is the viral form of M13. The single-stranded vectors are extruded through the cell membrane into the supernatant and recovered.

A synthetic oligonucleotide primer is then annealed to the single-stranded vectors containing the cDNA sequence in either the (+) or the (-) orientation by mixing in a buffered solution. The (+) orientation refers to the orientation in which the message-synonymous strand of the cDNA appears in the single-stranded vector. The (-) orientation refers to the orientation in which the coding strand of the cDNA, i.e., the complement of the (+) orientation, appears in the single-stranded vector. The manipulations discussed below apply equally well to vectors in either the (+) or the (-) orientation, although only those applicable to vectors in the (+) orientation are described in detail. For vectors in the (-) orientation, the complements of the primer would be used.

The primer comprises three consecutive nucleotides, i.e., a triplet, encoding aspartic acid and sufficient nucleotides on each side of the triplet that are complementary to the message-synonymous single-stranded cDNA to enable the primer to anneal to the single stranded cDNA at the position in which the triplet encoding aspartic acid corresponds to the triplet encoding amino acid position 31 of human proinsulin.

Nucleotide sequences forming triplets encoding aspartic acid are known. These include triplets having the base sequerces ATC and GTC (wherein C refers to cytosine, T refers to thymine, A refers to adenosine and G refers to guanine) read in the 5'→3' direction. The nucleotide sequence of a cDNA sequence encoding human proinsulin is also known.

The primer further comprises nucleotides on each side the aspartic acid encoding triplet which are complementary to the nucleotides of the message-synonymous cDNA on each side of the triplet encoding the amino acid arginine at amino acid position 31 of human proinsulin. The number of nucleotides in each of these sequences is sufficient to effectively anneal the primer to the message-synonymous cDNA. Typically, this requires at least three complementary nucleotides on each side of the aspartic acid encoding triplet.

The presently preferred primer contains six complementary nucleotides on each side of the triplet encoding aspartic acid. The base sequence of the preferred primer is CTCCCGXXXGGTCTT wherein XXX may be ATC or GTC. The nucleotide sequence having the base sequence GGTCTT is complementary to the portion of the message-synonymous cDNA strand for encoding amino acid positions 29 and 30 of human proinsulin and the nucleotide sequence having the base sequence CTCCCG is complementary to the portion of the message-synonymous cDNA strand for encoding amino acid positions 32 and 33 of human proinsulin.

The triplet having the base sequence ATC is presently preferred as the aspartic acid encoding triplet. It is preferred because it forms, with adjacent nucleotides, the Pvu I restriction enzyme recognition sequence having the base sequence CGATCG which is useful in identifying the altered sequence following cloning.

A primer containing the triplet having the base sequence GTC may also be used but requires that some identifiable factor such as a restriction enzyme recognition sequence also be annealed to the DNA template.

After the primer and, if necessary, the restriction enzyme recognition sequence have been annealed to the message-synonymous cDNA, the annealed sequence is extended using DNA polymerase. This is accomplished by mixing in a buffered solution the single-stranded vector with the annealed DNA sequences with sufficient deoxyguanosine 5'-triphosphate (dGTP), deoxyadenosine 5'-triphosphate (dATP), deoxycytidine 5'-triphosphate (dCTP) and deoxythymidine 5'-triphosphate (dTTP). DNA ligase is also added, to ligate the ends of the annealed DNA sequences once they have been extended. This forms a circular double-stranded vector.

The double-stranded vector is then introduced into E. coli cells, again using a standard technique. A very small percentage of E. coli cells are actually inoculated with the vector. The cells are spread out on an agar gel and allowed to grow. The double-stranded vector is

thereby allowed to replicate. Relication produces two groups of double-stranded vectors, one group containing cDNA encoding proinsulin precursors comprising normal human proinsulin and a second group containing altered cDNA encoding modified proinsulin precursors comprising human proinsulin with aspartic acid substituted for arginine at amino acid position 31.

E. coli cells containing the M13 vector grow at a slower rate than those cells not containing the vector. As the cells grow, plaques, i.e., "holes" in the growing cell population, are formed where the cells containing the M13 vectors are replicating. Each plaque is a substantially pure population having only one form of the cDNA, i.e., either encoding proinsulin precursors comprising normal human proinsulin or encoding the modified proinsulin precursors.

To acertain if cells from a particular plaque contain vectors having the altered cDNA, vectors from a portion of the cells of the particular plaque are digested with the restriction enzyme that recognizes the Pvu I site. The vectors having the restriction enzyme recognition sequence will be cleaved by the restriction enzyme, thereby forming non-circular DNA fragments. The vectors not containing the recognition sequence will remain intact. Identification of the digestion products is then made, e.g., by electrophoresis on an agarose gel.

E. coli cells identified as containing M13 vectors having the altered cDNA sequence are cultured in liquid media. Single-stranded vectors containing the altered cDNA are extruded through the cell wall into the supernatant and recovered.

To produce a modified proinsulin precursor having methionine substituted for arginine at amino acid position

65 in addition to aspartic acid substituted for arginine at amino acid position 31, a nucleotide triplet encoding methionine is substituted for the naturally-occurring nucleotide triplet encoding arginine at amino acid position 65 of human proinsulin in the message-synonymous cDNA previously altered to encode aspartic acid at amino acid position 31.

To effect this substitution, a second primer is annealed to a single-stranded vector containing the previously altered cDNA in the (+) orientation. The primer contains the triplet having the base sequence CAT, which encodes the amino acid methionine, plus sufficient nucleotides on each side of the triplet that are complementary to the message-synonymous cDNA template to enable the primer to be effectively annealed to the cDNA template so that the methionine encoding triplet encodes the amino acid position 65 of human proinsulin.

The nucleotide sequence of the preferred primer contains the base sequence AATGCCCATCTTCTG wherein the nucleotide sequence having the base sequence CTTCTG is complementary to the portion of the message-synonymous cDNA template encoding amino acid positions 63 and 64 of proinsulin and the nucleotide sequence having the sequence AATGCC is complementary to the portion of the message-synonymous cDNA template encoding amino acid positions 66 and 67 of proinsulin.

The preferred nucleotide sequence of the primer is particularly suitable because it contains the Mbo II restriction enzyme recognition sequence having the base sequence TCTTC enabling later identification of the modified vector.

The annealed sequence is extended and ligated as previously described, yielding a circular double-stranded vector. The double-stranded vector is again introduced into E. coli cells and allowed to replicate. Again, plaques form from those cells containing the M13 vector.

To identify double-stranded vectors containing modified cDNA sequence encoding both amino acid substitutions, vectors from cells of a particular plaque are digested with the restriction enzyme that recognizes the Pvu I site and the restriction enzyme recognizing the Mbo II site. The double-stranded cDNA sequence having both recognition sequences is cleaved by both restriction enzymes. Modified cDNA sequences encoding both amino acid substitutions are identified by electrophoresis on an agar gel.

Once the vectors containing the double-stranded cDNA sequence encoding modified proinsulin precursors having both substitutions have been identified, E. coli cells containing those vectors are cultured. Double-stranded vectors are then separated from the cells and the vectors are cleaved with restriction endonucleases to yield the altered cDNA fragment. The altered cDNA fragment is then isolated.

A second vector, known to express high levels of proinsulin in a production cell, such as the tryptophan fusion vector containing cDNA encoding human proinsulin is also cleaved by the same endonuclease to remove the natural cDNA. The second vector is then isolated.

The altered cDNA sequence isolated from the M13 vector is then inserted into the second vector. The second vector is then inserted into production cells and allowed to replicate. Cells containing the second vector are separated and cultured to produce a population expressing high levels of modified proinsulin precursors.

- 14 -

0090433

A preferred general method of isolating the modified proinsulin precursor comprises preparing a monoclonal antibody against a particular fragment of the modified proinsulin precursor, for example, the sequence from position 32 to 48 of the C chain. A synthetic peptide having such a suitable sequence is then prepared. Mice are then immunized with the peptide and monoclonal antibody-secreting cells are recovered by the process of polyethylene glycol-mediated cell fusion. Cells secreting monoclonal antibodies against the particular human proinsulin fragment are identified by sandwich radioimmunoassay. Monoclonal antibodies are purified from the supernatants of the culture fluid, and attached to chromatographic agarose beads to generate a matrix having a specific affinity for that human proinsulin fragment.

The modified proinsulin precursors elaborated by the production cell of interest are isolated by specific absorption to the monoclonal antibody column. A preparation of the modified proinsulin precursor is concentrated and partially purified by precipitation with ammonium sulfate added in an amount empirically determined to precipitate the modified proinsulin precursor. The partially purified preparation is then passed through the monoclonal antibody column, and the column is washed to remove unabsorbed contaminant proteins. Modified proinsulin precursors are then specifically desorbed by passing an ammonium carbonate buffer of pH approximately 10 and ionic strength of about 0.1 through the column.

Modified proinsulin precursors that have been isolated are then cleaved at the carboxyl terminus of the C chain, i.e. between position 65 and 66, and, if necessary, at the amino terminus of the B chain, i.e., position -1, by treatment with cyanogen bromide. Modified proinsulin precursors are freed from extraneous salts by dialysis and lyophilization and then dissolved in pure formic acid, diluted to about 70% with water, and cleaved with 100 to 200 fold excess of cyanogen bromide over methionyl residues. About 5 mg of modified proinsulin precursor are dissolved in each ml of formic acid. The reaction proceeds for about 24 hours at room temperature in the dark, after which the cleavage products are lyophilized to remove excess reagent.

Cleavage at the amino terminus of the C chain, i.e., between amino acid position 31 and 33 and, if necessary, at the carboxyl terminus of the A chain is affected by dissolving the modified proinsulin precursor in about 0.01 M ammonium bicarbonate (pH 8.0) containing 2 M urea. The modified proinsulin precursor is dissolved to a concentration of about 5 mg/ml, and cleavage is carried out by addition of P. fragi mutant Me 1 enzyme in about 1/100 the concentration of the modified proinsulin precursor (i.e., about 50 ug/ml). The reaction is allowed to proceed for about 24 hours at about 37°C.

Insulin liberated from cleaved modified proinsulin precursor is purified by conventional procedures.

As indicated, the above-described method works equally well when the coding strand of the cDNA appears in the single-stranded form of the vector. In this situation, the primers that are prepared and annealed to the vector are the complements of the above-described primers. For example, to effect the substitution of aspartic acid for arginine at amino acid position 31, the preferred primer that is annealed to the coding strand would have the base sequence AAGACCGATCGGGAG which is the complement of the primer having the base sequence CTCCCGATCGGTCTT which is annealed to the message-synonymous strand.

Likewise, a primer having the base sequence CAGAAGATGGGCATT is preferred to be annealed to the coding strand to effect the substitution of methionine to arginine at amino acid position 65. This primer is the complement of the above-described primer having the base sequence AATGCCCATCTTCTG.

In the production of a DNA sequence encoding modified proinsulin precursors, M13 is the presently preferred vector because of its unique ability to exist in both a double-stranded and a single-stranded form. This enables a primer to be annealed to the single-stranded viral form without first chewing back one strand.

However, a double-stranded vector or plasmid may be used. This requires that prior to annealing the synthetic primer to the cDNA present in the double-stranded vector on plasmid, one strand of the double-stranded cDNA must be chewed back. The nucleotide sequence of the primer is adjusted according to which strand of the cDNA is removed.

This process may be accomplished by cleaving the circular double-stranded vector or plasmid having a natural cDNA sequence encoding proinsulin. The cleavage is made at one end of the natural cDNA with an endonuclease. The cleaved plasmid or vector is then treated with an exonuclease to chew back, e.g., in the 5'→3' direction, one strand of the double-stranded cDNA starting at the cleaved end. The exonuclease must chew back one strand of the cDNA sufficiently to provide a single-stranded nucleotide sequence of sufficient length to enable the synthetic primer to be annealed to the single-stranded portion of the cDNA at the appropriate position.

The synthetic primer is then annealed to the single-stranded portion of the cDNA, extended, and both strands ligated to form a circular double-stranded vector or plasmid, which is then introduced into E. coli and allowed to replicate. Subsequent procudures are as previously described for M13.

The disadvantage of this method is the difficulty in knowing when to terminate the action of the exonuclease which tends to reduce the efficiency and reliability of this method.

It is presently preferred to anneal a synthetic primer encoding an amino acid substitution at amino acid position 31 of proinsulin followed by annealing a primer encoding an amino acid substitution at amino acid position 65 to the single-stranded M13 vector having natural cDNA. However, if both substitutions are desired, both primers may be annealed simultaneously rather than in sequence. However, there may be a disadvantage in annealing the two primers simultaneously because of the probability of achieving the desired result.

The success rate for recovering an altered cDNA sequence can be as low as 1 in 20. Isolation and identification of a plaque having cells displaying the desired altered cDNA in a subsequently grown colony may be accomplished relatively quickly as at least about 1 in every 20 plaques will have the desired DNA alternation.

Simultaneous annealing of two synthetic primers may result in a success rate of as low as 1 in 400. This could require isolation and identification of about 400 such plaques to find one with the desired characteristics. Accordingly, detection of such a plaque requires relatively lengthy analysis.

The previously described method of isolation and separation using monoclonal antibodies is presently preferred as a general method.

Another preferred method for recovery of modified proinsulin precursors expressed as E. coli tryptophan operon fusion peptides exploits the high insolubility of proteins (including human proinsulin) containing tryptophan operon peptide amino termini. The insoluble fusion proteins are easily purified from lysed E. coli cells by differential centrifugation in the presence of physiological salt concentrations (0.1 to 0.2 molar NaCl). Other conventional techniques, such as conventional ion exchange and gel exclusion chromotography, may be used successfully.

An alternate preferred embodiment for cleavage of the C chain of the modified proinsulin precursors is particularly utile if certain bacteria and yeasts are used as production cells. Certain cells comprise the capability of cleaving the initial amino acid chain preceding the B chain amino terminus of proinsulin.

If such production cells are used, the cDNA is altered to provide a modification of human proinsulin at only the amino terminus of the C chain wherein aspartic acid is substituted for arginine. No substitution is made at the carboxyl terminus of the C chain. The procedure for effecting the substitution at the amino terminus of the C chain and for isolating the proinsulin thus modified is the same as previously described.

Cleavage of the C chain to form insulin may be accomplished by digestion with P. fragi mutant enzyme as previously described for about 24 hours. This cleaves between amino acid positions 30 and 31 of the modified proinsulin precursor and, if necessary, between the carboxyl terminus of the A chain and the amino terminus of an amino acid chain following the A chain. After about 24 hours, trypsin is added in about 1/1000 of the concentration of the modified proinsulin precursor. Digestion is allowed to continue for a predetermined length of time, typically about 2 hours. The trypsin digestion cleaves between amino acid positions 65 and 66. The reaction is stopped by rapid cooling or by addition of an inhibitor such as soybean trypsin inhibitor, diisopropyl-fluorophosphate, or p-toluenesulfonyl fluoride.

Insulin produced by this method is purified by conventional procedures such as solvent fractionation, salt precipitation, specific absorption on a monoclonal antibody column and the like.

WHAT IS CLAIMED IS:

1. A modified proinsulin precursor comprising proinsulin containing neither methionine nor aspartic acid in either the A or B chains and

    a. aspartic acid substituted for the amino acid at the amino terminus of the C chain; or

    b. methioni. substituted for the amino acid at the carboxyl terminus of the C chain; or

    c. aspartic acid substituted for the amino acid at the amino terminus of the C chain and methionine substituted for the amino acid at the carboxyl terminus of the C chain.

2. A modified proinsulin precursor comprising human proinsulin wherein:

    a. aspartic acid is substituted for arginine at amino acid positon 31; or

    b. methionine is substituted for arginine at amino acid position 65; or

    c. aspartic acid is substituted for arginine at amino acid position 31 and methionine is substituted for arginine at amino acid position 65.

3. A DNA sequence encoding a modified proinsulin precursor comprising a DNA sequence encoding proinsulin wherein:

a. a nucleotide triplet encoding aspartic acid is substituted for the nucleotide triplet encoding the amino acid at the amino terminus of the C chain; or

b. a nucleotide triplet encoding methionine is substituted for the nucleotide triplet encoding the amino acid at the carboxyl terminus of the C chain; or

c. a nucleotide triplet encoding aspartic acid is substituted for the nucleotide triplet encoding the amino acid at the amino terminus of the C chain and a nucleotide triplet encoding methionine is substituted for the nucleotide triplet encoding the amino acid at the carboxyl terminus of the C chain.

4. A DNA sequence as claimed in claim 3 wherein the nucleotide triplet encoding aspartic acid comprises a base sequence ATC.

5. A DNA sequence as claimed in claim 3 wherein the nucleotide triplet encoding aspartic acid comprises a base sequence GTC.

6. A DNA sequence as claimed in claim 3 wherein the nucleotide triplet encoding methionine comprises a base sequence CAT.

7. A DNA sequence encoding a modified proinsulin precursor comprising a DNA sequence encoding human proinsulin wherein:

a. a nucleotide triplet comprising a base sequence selected from the group consisting of ATC and GTC is substituted for the nucleotide triplet encoding arginine at amino acid position 31; or

b. a nucleotide triplet comprising a base sequence CAT is s ituted for the nucleotide triplet encoding arginine . amino acid position 65; or

c. a nucleotide triplet comprising a base sequence selected from the group consisting of ATC and GTC is substituted for the nucleotide triplet encoding arginine at amino acid position 31 and a nucleotide triplet comprising a base sequence CAT is substituted for the nucleotide triplet encoding arginine at amino acid position 65.

8. A process for the production of DNA sequences encoding modified proinsulin precursor having aspartic acid at the amino terminus of the C chain comprising:

a. preparing a primer comprising a nucleotide triplet encoding aspartic acid and sufficient nucleotides on each side of the triplet that are complementary to nucleotides of a single-stranded, message-synonymous DNA sequence encoding a proinsulin precursor comprising proinsulin to enable the primer to be effectively annealed to the single-stranded DNA sequence at a position wherein the triplet encoding aspartic acid encodes the amino acid at the amino terminus of the C chain of the proinsulin precursor;

b. annealing the primer to a vector containing the single-stranded, message-synonymous DNA sequence encoding the proinsulin precursor;

c. extending and ligating the primer to form a circular double-stranded vector;
vector; and

e. allowing the cells and the double-stranded vector to replicate, thereby forming at least a first group of vectors comprising unaltered DNA sequences encoding the proinsulin precursors and a second group of vectors comprising altered DNA sequences encoding modified proinsulin precursors containing aspartic acid at the amino terminus of the C chain.

9. A process as claimed in claim 8 further comprising preparing and annealing a nucleotide sequence comprising a restriction enzyme recognition sequence to the single-stranded DNA sequence of the vector.

10. A process as claimed in claim 8 wherein the synthetic primer comprises a nucleotide sequence having a restriction enzyme recognition sequence.

11. A process as claimed in claim 8 wherein the nucleotide triplet encoding aspartic acid has a base sequence of ATC.

12. A process as claimed in claim 8 wherein the proinsulin presurcors comprise human proinsulin and the primer comprises a nucleotide sequence having a base sequence CTCCCGATCGGTCTT.

13. A process as claimed in claim 8 further comprising:

a. preparing a second primer comprising a nucleotide triplet encoding methionine and sufficient nucleotides on each side of the triplet that are complementary to the message-synonymous strand of the altered DNA sequence to enable the second primer to be effectively annealed to the message-synonymous strand of the altered DNA sequence at a position wherein the nucleotide triplet encoding methionine encodes the amino terminus of the C chain of the proinsulin;

b. providing from the second group of vectors, a vector comprising the message-synonymous strand of the altered DNA sequence;

c. annealing the second primer to the message-synonymous strand of the altered DNA sequence;

d extending and ligating the second primer to form a second circular double-stranded vector;

e. transforming cells with the second double stranded vector; and

f. allowing the cells and the second double-stranded vector to replicate to thereby form at least a third group of vectors comprising the previously altered DNA sequence and a fourth group of vectors comprising a twice-altered DNA sequence encoding modified proinsulin precursors comprising human proinsulin having aspartic acid at the amino terminus of the C chain and methionine at the carboxyl terminus of the C chain.

14. A process as claimed in claim 13 wherein a nucleotide sequence comprising a second restriction enzyme recognition sequence is prepared and annealed to the message-synonymous strand of the altered DNA sequence.

15. A process as claimed in claim 13 wherein the second primer comprises a nucleotide sequence having a restriction enzyme recognition sequence.

16. A process as claimed in claim 13 wherein the nucleotide triplet encoding methionine has a base sequence CAT.

17. A process as claimed in claim 13 wherein the proinsulin precursors comprise human proinsulin and the second primer comprises a nucleotide sequence having a base sequence AATGCCCATCTTCTG.

18. A process for the production of DNA sequences encoding modified proinsulin precursor having aspartic acid at the amino terminus of the C chain comprising:

a. preparing a primer comprising a nucleotide triplet that is complementary to a nucleotide triplet encoding aspartic acid and sufficient nucleotides on each side of the triplet that are complementary to nucleotides of a single-stranded coding DNA sequence encoding proinsulin precursors comprising proinsulin to enable the primer to be effectively annealed to the coding DNA sequence so that the triplet that is complementary to a triplet encoding aspartic acid is at the position of the complement of the triplet that encodes the amino acid at the amino terminus of the C chain of the proinsulin;

b. annealing the primer to a vector containing the single-stranded coding DNA sequence encoding the proinsulin precursor;

c. extending and ligating the primer to form a circular double-stranded vector;

d. transforming cells with the double-stranded vector; and

e. allowing the cells and the double-stranded vector to replicate, thereby forming at least a first group of vectors comprising unaltered DNA sequences encoding the proinsulin precursor and a second group of vectors comprising altered DNA sequences encoding modified proinsulin precursors containing aspartic acid at the amino terminus of the C chain.

0090433

19. A process as claimed in claim 18 further comprising preparing and annealing a nucleotide sequence comprising a nucleotide sequence that is complementary to a restriction enzyme recognition sequence to the single-stranded coding DNA sequence.

20. A process as claimed in claim 18 wherein the synthetic primer comprises a nucleotide sequence that is complementary to a restriction enzyme recognition sequence.

21. A process as claimed in claim 18 wherein the nucleotide triplet that is complementary to a nucleotide triplet encoding aspartic acid has a base sequence GAT.

22. A process as claimed in claim 18 wherein the proinsulin precursors comprise human proinsulin and the primer comprises a nucleotide sequence having a base sequence AAGACCGATCGGGAG.

23. A process as claimed in claim 18 further comprising:

a. preparing a second primer comprising a nucleotide triplet that is complementary to a nucleotide triplet encoding methionine and sufficient nucleotides on each side of the triplet that are complementary to nucleotides of the coding strand of the altered DNA sequence to enable the second primer to be effectively annealed to the coding strand of the altered DNA sequence so that the nucleotide triplet that is complementary to a nucleotide triplet encoding methionine is at the position of the complement of the triplet that encodes the amino acid at the amino terminus of the C chain of the proinsulin;

b. providing from the second group of vectors, a vector comprising the coding strand of the altered DNA sequence;

c. annealing the second primer to the coding strand of the altered DNA sequence;

d. extending and ligating the second primer to form a second circular double-stranded vector;

e. transforming cells with the second double-stranded vector; and

f. allowing the cells and the second double-stranded vector to replicate to thereby form at least a third group of vectors comprising the previously altered DNA sequence and a fourth group of vectors comprising twice-altered DNA sequences encoding modified proinsulin precursors comprising human proinsulin having aspartic acid at the amino terminus of the C chain and methionine at the carboxyl terminus of the C chain from the fourth group of vectors.

24. A process as claimed in claim 23 wherein a nucleotide sequence comprising a nucleotide sequence that is complementary to a second restriction enzyme recognition sequence is prepared and annealed to the coding strand of the altered DNA sequence.

25. A process as claimed in claim 23 wherein the second primer comprises a nucleotide sequence that is complementary to a restriction enzyme recognition sequence.

26. A process as claimed in claim 23 wherein the nucleotide triplet that is complementary to a nucleotide triplet encoding methionine has a base sequence ATG.

27. A process as claimed in claim 23 wherein the proinsulin precursors comprise human proinsulin and the second primer comprises a nucleotide sequence having a base sequence CAGAAGATGGGCATT.

28. A process for the production of a DNA sequence encoding a modified proinsulin precursor comprising:

a. preparing a first primer comprising a nucleotide triplet encoding aspartic acid and sufficient nucleotides on each side of the triplet that are complementary to nucleotides of a single-stranded, message-synonymous DNA sequence encoding proinsulin precursors comprising a proinsulin to enable the first primer to be effectively annealed to the single-stranded DNA sequence at a position wherein the triplet encoding aspartic acid encodes the amino acid at the amino terminus of the C chain of the proinsulin;

b. preparing a second primer comprising a nucleotide triplet encoding methionine and sufficient nucleotides on each side of the triplet that are complementary to nucleotides of the single-stranded, message-synonymous DNA sequence to enable the second primer to be effectively annealed to the single-stranded DNA sequence at a position wherein the triplet encoding methionine encodes the amino acid at the carboxyl terminus of the C chain of the proinsulin;

c. annealing the first primer and second primer to a vector containing the single-stranded, message-synonymous DNA sequence encoding the proinsulin precursor;

d. extending and ligating the primers to form a circular double-stranded vector;

e. transforming a cell with the double-stranded vector; and

f. allowing the cell and the double-stranded vector to replicate to thereby form at least a first group of vectors comprising unaltered DNA sequences encoding proinsulin precursors and a second group of vectors comprising altered DNA sequences encoding modified proinsulin precursors containing aspartic acid at the amino terminus of the C chain and methionine at the carboxyl terminus of the C chain.

29. A process as claimed in claim 28 wherein at least one nucleotide sequence comprising a restriction enzyme recognition sequence is also prepared and annealed to the single-stranded DNA sequence encoding the proinsulin.

30. A process as claimed in claim 28 wherein the first primer comprises a nucleotide sequence having a restriction enzyme recognition sequence.

31. A process as claimed in claim 28 wherein the second primer comprises a nucleotide sequence having a restriction enzyme recognition sequence.

32. A process as claimed in claim 28 wherein the first primer and the second primer each comprise a nucleotide sequence having a restriction enzyme recognition sequence.

33. A process as claimed in claim 28 wherein the nucleotide triplet encoding aspartic acid has a base sequence ATC.

34. A process as claimed in claim 28 wherein the proinsulin precursors comprise human proinsulin and the first primer comprises a nucleotide sequence having a base sequence CTCCCGATCGGTCTT.

35. A process as claimed in claim 28 wherein the nucleotide triplet encoding methionine has a base sequence CAT.

36. A process as claimed in claim 28 wherein the proinsulin precursors comprise human proinsulin and the second primer comprises a nucleotide sequence having a base sequence AATGCCCATCTTCTG.

37. A process for the production of a DNA sequence encoding a modified proinsulin precursor comprising:

a. preparing a first primer comprising a nucleotide triplet that is complementary to a nucleotide triplet encoding aspartic acid and sufficient nucleotides on each side of the nucleotide triplet that are complementary to nucleotides of the coding strand of a DNA sequence encoding proinsulin precursors comprising proinsulin to enable the first primer to be effectively annealed to the coding strand of the DNA sequence so that the nucleotide triplet that is complementary to a nucleotide triplet encoding aspartic acid is at the position of the complement of the nucleotide triplet that encodes the amino acid at the amino terminus of the C chain of the proinsulin;

b. preparing a second primer comprising a nucleotide triplet that is complementary to a nucleotide triplet encoding methionine and sufficient nucleotides on each side of the nucleotide triplet that are complementary to nucleotides of the coding strand of the DNA sequence encoding the proinsulin precursors to enable the second primer to be effectively annealed to the coding strand of the DNA sequence so that the nucleotide triplet that is complementary to a nucleotide triplet encoding methionine is at the position of the complement of the nucleotide triplet that encodes the amino acid at the carboxyl terminus of the C chain of the proinsulin;

c. annealing the first primer and the second primer to a vector containing the coding strand of the DNA sequence encoding the proinsulin precursors;

d. extending and ligating the primers to form a circular double-stranded vector;

e. transforming a cell with the double-stranded vector; and

f. allowing the cell and the double-stranded vector to replicate to thereby form at least a first group of vectors comprising unaltered DNA sequences encoding proinsulin precursors and a second group of vectors comprising altered DNA sequences encoding modified proinsulin precursors containing aspartic acid at the amino terminus of the C chain and methionine at the carboxyl terminus of the C chain.

38. A process as claimed in claim 37 wherein at least one nucleotide sequence comprising a nucleotide sequence that is complementary to a restriction enzyme recognition sequence is also prepared and annealed to the coding strand of the DNA sequence.

39. A process as claimed in claim 37 wherein the first primer comprises a nucleotide sequence that is complementary to restriction enzyme recognition sequence.

40. A process as claimed in claim 37 wherein the second primer comprises a nucleotide sequence that is complementary to a restriction enzyme recognition sequence.

41. A process as claimed in claim 37 wherein the first primer and the second primer each comprise a nucleotide sequence that is complementary to a restriction enzyme recognition sequence.

42. A process as claimed in claim 37 wherein the nucleotide triplet that is complementary to a nucleotide triplet encoding aspartic acid has a base sequence GAT.

43. A process as claimed in claim 37 wherein the proinsulin precursors comprise human proinsulin and the first primer comprises a nucleotide sequence having a base sequence AAGACCGATCGGGAG.

44. A process as claimed in claim 37 wherein the second primer comprises a nucleotide triplet having a base sequence ATG.

45. A process as claimed in claim 18 wherein the proinsulin precursors comprise human proinsulin and the second primer comprises a nucleotide sequence having a base sequence CAGAAGATGGGCATT.

46. A process for the preparation of insuline comprising:

a. preparing a DNA sequence encloding a modified proinsulin precursor as described in claim 3;

b. inserting the DNA sequence into a first vector;

c. transforming a cell with the first vector;

d. isolating modified proinsulin precursors elaborated by the cell; and

e. cleaving the modified proinsulin precursors to yield insulin.

47. A process as claimed in claim 46 wherein the modified proinsulin precursors comprise aspartic acid at the amino terminus of the C chain and are cleaved between the carboxyl terminus of the B chain and the amino terminus of the C chain by digestion with a sufficient amount of P. fragi mutant Me 1 enzyme.

48. A process as claimed in claim 46 wherein the modified proinsulin precursors comprise aspartic acid at the amino terminus of the C chain and at the carboxyl terminus of an amino acid chain following the A chain and are cleaved between the carboxyl terminus of the B chain and the amino terminus of the A chain and between the carboxyl terminus of the amino acid chain following the A chain and the amino terminus of the A chain by digestion with a sufficient amount of P. fragi mutant Me 1 enzyme.

49. A process as claimed in claim 47 to 48 wherein the modified proinsulin precursors further comprise methionine at the carboxyl terminus of the C chain and are cleaved between the C chain and the A chain by digestion with a sufficient amount of trypsin.

50. A process as claimed in claim 46 wherein the modified proinsulin precursors comprise methionine at the carboxyl terminus of the C chain and are cleaved between the amino terminus of the C chain and the carboxyl terminus of the A chain by treatment with an aqueous solution comprising cyanogen bromide in a concentration sufficient to thus cleave the modified proinsulin precursors.

51. A process as claimed in claim 46 wherein the modififed proinsulin presursors comprise methionine at the carboxyl terminus of the C chain and at the carboxyl terminus of an amino acid sequence attached to the amino terminus of the B chain and are cleaved between the amino terminus of the B chain and the carboxyl terminus of the amino acid sequence attached to the amino terminus of the B chain by treatment with cyanogen bromide in a concentration sufficient to thus cleave the modifired proinsulin precursors.

## DOCUMENTS CONSIDERED TO BE RELEVANT

EP 83103253.7

| Category | Citation of document with indication, where appropriate, of relevant passages | Relevant to claim | CLASSIFICATION OF THE APPLICATION (Int. Cl. 3) |
|---|---|---|---|
| A,P | EP - A2 - 0 070 632 (CETUS COR-PORATION) (26-01-1983) <br> * Claims 1,10 * | 3,7,8 | C 12 N 15/00 <br> C 12 P 21/02 <br> C 07 C 103/42 <br> C 12 P 19/34 <br> C 07 H 21/00 |
| A,P | EP - A2 - 0 068 701 (CANADIAN PATENTS AND DEVELOPMENT LIMITED) (05-01-1983) <br> * Abstract * | 3,7,8 | |
| A,P | EP - A2 - 0 055 945 (GENENTECH, INC.) (14-07-1982) <br> * Abstract; claims 9,19 * | 3,7,8, 46 | |
| A | EP - A1 - 0 026 598 (THE REGENTS OF THE UNIVERSITY OF CALIFORNIA) <br> * Claims 1-5,12 * | 3,7,8 | |
| A | GB - A - 2 067 574 (HOECHST AKTIENGESELLSCHAFT) <br> * Abstract * | 3,7,8 | TECHNICAL FIELDS SEARCHED (Int. Cl. 3) <br><br> C 12 N <br> C 12 P <br> C 07 C 103/00 <br> C 07 H |
| A | GB - A - 2 007 675 (GENENTECH, INC.) <br> * Claim 12 * | 3,7,8 | |

The present search report has been drawn up for all claims

| Place of search <br> VIENNA | Date of completion of the search <br> 17-06-1983 | Examiner <br> WOLF |
|---|---|---|

| | DOCUMENTS CONSIDERED TO BE RELEVANT | | CLASSIFICATION OF THE APPLICATION (Int. Cl.) |
|---|---|---|---|
| Category | Citation of document with indication, where appropriate, of relevant passages | Relevant to claim | |
| A | SCIENCE, vol. 208, April 4, 1980, USA<br><br>I. SURES et al. "Nucleotide Sequence of Human Preproinsulin Complementary DNA" pages 57-59<br><br>   * Totality *<br><br>---- | 3,7,8 | |
| | | | TECHNICAL FIELDS SEARCHED (Int. Cl.) |

EPO Form 1503.2   06.78